# EUROPEAN PATENT SPECIFICATION

(11) **EP 0 483 150 B1**
(45) Date of publication and mention of the grant of the patent: **11.10.1995**
(21) Application number: 90905407.4
(22) Date of filing: 04.04.1990
(51) Int. Cl.: A61H 39/02, A61H 39/00

(54) **ACUPUNCTURE LOCATING DEVICE**
VORRICHTUNG ZUM LOKALISIEREN VON AKUPUNKTURPUNKTEN
DISPOSITIF DE LOCALISATION POUR L'ACUPUNCTURE

(30) Priority: 05.04.1989 CA 595819
(43) Date of publication of application: 06.05.1992
(73) Proprietor: ISHIKAWA, Keihachi, Ontario K1S 5H8 (CA)
(72) Inventor: ISHIKAWA, Keihachi, Ontario K1S 5H8 (CA)
(74) Representative: Gardi, Giuliano
(86) International application number: PCT/CA90/00110
(87) International publication number: WO 90/11751

(56) References cited:
- DE-A- 2 533 507
- US-A- 3 901 214
- US-A- 4 052 978

## Description

### TECHNICAL FIELD

This invention relates to apparatus for determining cutinous points and capable of providing electrical treatment to parts of an animal body. The apparatus is particularly applicable for use with a human body.

Acupuncture is becoming increasingly recognized in the western world for the treatment of certain problems which can occur in the human body. The word "acupuncture" refers to the introduction of needles into body tissue at certain points which have a very small diameter. These points will be referred to herein as "cutinous points."

In acupuncture work it is particularly important to determine cutinous points on a patient's body so that treatment can be applied to the correct points thereof. Previously, apparatuses intended to serve this purpose have been provided, such as, for example, that disclosed in United States patent 4,052,978 (Eugenio), but these have been relatively large and cumbersome.

### DISCLOSURE OF THE INVENTION

It is an object of the present invention to provide apparatus for determining cutinous points which is not so combersome as apparatus previously known to the inventor.

According to the present invention there is provided an apparatus for determining cutinous points in an animal body. In a preferred embodiment, the device is adapted to also provide electrical treatment in the form of pulse current to the body. The device is comprised of two metallic terminals for making contact with the skin of said body, said terminals forming part of an electrical circuit, the first of said two terminals being connected through a first resistor to the first base electrode of a transistor, a second electrode of said transistor being connected to one end of the primary winding of a transformer, a third electrode of said transistor being connected to one side of a battery, the other side of the battery being connected to an intermediary tapping point of said transformer, the second said terminal being connected directly or indirectly to the opposite end of said primary winding, a capacitor connected between said base electrode and said second terminal, and an indicator means connected across a secondary of said transformer.

### BRIEF DESCRIPTION OF DRAWINGS

Some embodiments of the invention will now be described, by way of example, with reference to the accompanying drawings in which:-
Figure 1 is a diagrammatic representation of a circuit for use in apparatus according to one embodiment of the present invention,
Figure 2 is a graphical representation of the voltage waveform at certain points in the circuit of Figure 1,
Figure 3 is a perspective view of apparatus constructed according to an embodiment of the invention,
Figure 4 is an end view of the apparatus of Figure 3,
Figure 5 is a perspective view of apparatus constructed according to another embodiment of the invention.

### BEST MODE FOR CARRYING OUT THE INVENTION

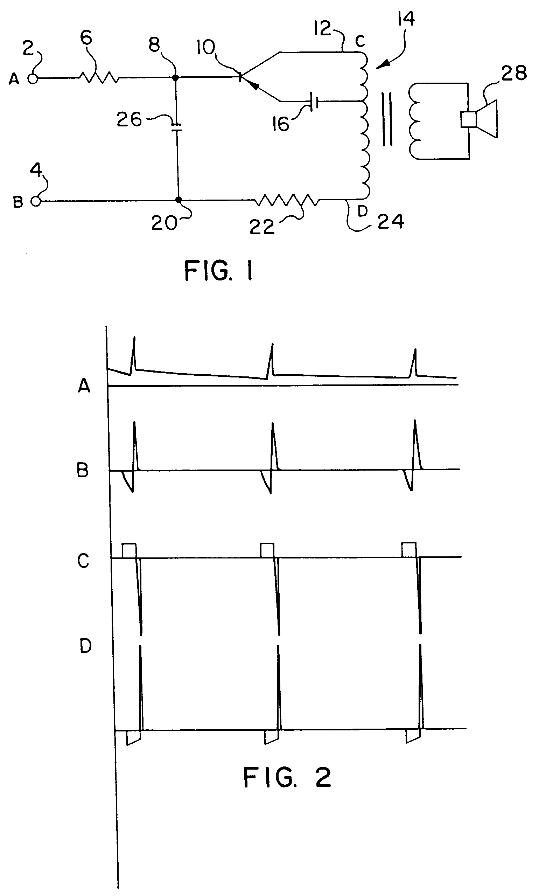

Referring to Figure 1 the circuit comprises two terminals 2, 4 for making contact with the skin of a human being, said terminals forming part of an electrical circuit. The first of said two terminals 2 is connected through a first resistor 6 and through an intermediary point 8 to the base electrode of a transistor 10, another electrode of said transistor being connected to one end 12 of the primary winding of a transformer 14, and a third electrode being connected to one side of a battery 16. The other side of the battery 16 is connected to an intermediate tapping point 18 of the said transformer 14.

The second terminal 4 is connected through an intermediary point 20 and a second resistor 22 to the opposite end 24 of said primary winding.

A capacitor 26 is connected between said intermediary points 8 and 20.

A loudspeaker 28 is connected across a secondary of said transformer 14. The waveforms in Figure 2 are illustrated for the purpose of understanding the operation of the circuit of Figure 1 as described below.

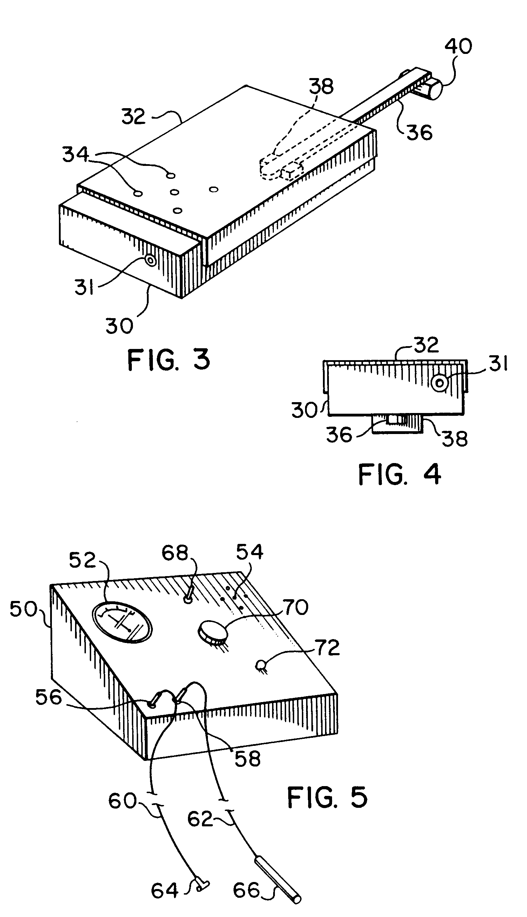

The perspective view of the embodiment illustrated in Figure 3 shows a unit capable of being held in the band of the doctor or the patient for determining the relevant cutinous points of the patient's body or for applying electrical treatment to a part of the patient's body. If the device is held by the doctor, the free hand of the doctor or some other part of the doctor, should make contact with the skin of the patient to complete an electrical circuit. Alternately a cord may be plugged into socket 31 (Figures 3 and 4). A metallic probe is connected to the other end of the cord and held by the patient.

The unit comprises a rectanguloid body 30 of insulating material, e.g. plastic, containing the components of the electrical circuit of Figure 1. A metallic U-shaped plate member 32 is affixed over part of the body 30 and is connected to terminal 2 in the circuit of Figure 1. Five holes 34 are provided through the top surface of body 30 and plate member 32 whereby sound from loudspeaker 28 (Figure 1) can be heard.

A metallic extendible arm 36 projects beyond the insulating body 30 and is movable longitudinally, lateral movement being restricted by guide members such as 38 (Figures 3 and 4). A contact terminal member 40, of semicircular cross-section is integrally and electrically attached to the end of arm 36, the arm 36 having an electrical connection to terminal 4 in the circuit of Figure 1.

The device of Figures 3 and 4 includes the circuit of Figure 1, as stated above. The operation of the device will now be described with reference to Figures 1 through 4. It will be observed that Figure 2 is a graphical representation of the voltage waveforms at points A, B, C and D of Figure 1. In use, terminals 2 and 4 are applied to the skin of a patient and capacitor 26 is charged up. Current then ceases to flow until terminal 4 contacts a cutinous point of the patient. Such cutinous points are substantially more conductive than other parts of the skin, approximately 50 to 100 kilohms in most cases. The normal skin surface has a resistivity of around 10 to 20 megohms. Dub to the change in resistivity, capacitor 26 begins to discharge and when its voltage is lower than the supply voltage across terminals 8 and 20 by 0.6 to 0.7 volts, base current of transistor 10 starts to flow. This completes the circuit through part of the primary winding of transformer 14 back to the negative terminal battery 16. However the base current of transistor 10 charges the capacitor 26 back above its threshold and in effect turns the transistor off, causing the collector current to decrease to zero whereby transformer 14 generates a negative pulse at point C, as shown in Figure 2. The winding of transformer 14 is such that an inverted signal, is induced at point D. Therefore a positive pulse is produced which charges capacitor 26 through resistor 22 to an even higher voltage. This ensures the off state of transistor 10 is maintained. However, the passage of electricity through the patient's skin allows capacitor 26 to discharge and as soon as the voltage is below the threshold level the whole sequence is repeated. This generates an audible signal through the secondary winding of transformer 14 and miniature loudspeaker 28. The audible signal produced depends on the conductivity of the various locations at which the terminals 2 and 4 are located, terminal 2 normally being electrically connected to a metallic portion held in the hand of the patient whilst terminal 4 is connected to a metallic portion which is moved over the skin to determine a cutinous point. In the apparatus of Figure 3 terminal 4 is connected to the contact terminal member 40.

It is important to observe that point B is negative in relation to point A and a negative potential is applied in order to stimulate the cutinous points. When a cutinous point is detected and it is desired to stimulate that point, the metallic part corresponding to point B should be maintained in contact with the patient's skin at the respective cutinous point for a short period of time, normally 10 to 15 seconds. This appears to have substantial advantage over the method of using mechanical stimulation by means of a needle or heat, etc. It will be understood that with a reverse polarity stimulation will not take place although detection of the cutinous point on the patient's skin is still possible.

Referring to Figure 5, there is illustrated a further embodiment of the invention which comprises a housing 50 having a sloping upper surface on which is located an indicating meter 52, holes 54 therein for the emergence of sound from loudspeaker 28 of Figure 1, and plug-in terminal locations 56 and 58. The ends of wires 60 and 62 are connected in the terminals 56 and 58 whilst at the end of said wires are metallic probe portions 64 and 66 as illustrated in Figure 5. Thus the metallic portions 64 and 66 may be placed at any two separate locations on a patient's skin so as to determine cutinous points. It will be understood that the embodiments shown in Figure 5 are more convenient for use in a doctor's office whilst the embodiment of Figure 3 is convenient for use either in a doctor's office or by the patient alone. In this way stimulation of the skin may be achieved or the cutinous points determined for further treatment.

The embodiment of Figure 5 is also provided with an on-off switch 68, and adjustable current limiter 70 and a volume control 72.

The type and value of the different components can readily be selected by an expert skilled in the art. It will also be understood that instead of using loudspeaker 28 some other indicating means, such as a lamp or meter, may be used.

It will be understood that the component values in Figure 1 could readily be changed. If a battery of higher voltage was substituted for battery 16, then resistor 22 could be omitted and resistor 6 increased in value. Thus terminal 4 and transformer end 24 would be directly connected together instead of being indirectly connected through resistor 22. A lower current of 30 to 40 micro-amps could be achieved.

In Figure 3 the end of arm 36 and/or at least one end of contact terminal member 40 could be tapered to a point to facilitate use around parts of a patient's body, for example the ears. Arm 36 could, if desired, be made non-extendable but fixed in position.

## Claims

1. An apparatus for determining cutinous points within an animal body consisting of:
(a) two metallic, electrically conductive terminals (32, 40/64, 66) for making contact with the skin of said body;
(b) said terminals (32, 40/64, 66) forming part of an electrical circuit;
(c)
(i) the first (32/66) of said two terminals (32, 40/64, 66) being connected through a first resistor (6) to the first base elctrode of a transistor (10), said transistor adapted to generate a pulse current by the on/off swithcing thereof;
(ii) a second electrode of said transistor (10) being connected to one end (12) of the primary winding of a transformer (14);
(iii) a third electrode of said transistor (10) being connected to one side of a battery (16), the other side of the battery (16) being connected to an intermediary tapping point of said transformer (14);
(iv) the second (40/64) of said terminals being connected directly or by the intermediary of a resistor to the opposite end (24) of said primary winding;
(v) a capacitor (26) connected between said base electrode and said second terminal, which capacitor discharges at an increased rate as the electrical resistivity of the skin drops, thereby generating an increased pulse rate in said pulse current;
(vi) an indicator means (28) connected across a secondary winding of said transformer (14), said indicator means adapted to indicate the frequency of said pulse current.

2. An apparatus according to claim 1 adapted to provide electrical treatment to said body in the form of pulse current in therapeutic dosage strength.

3. An apparatus according to claims 1 or 2 wherein said indicator means (28) is an apparatus for emitting audible sound at the frequency of said pulse current.

4. An apparatus according to claims 1 or 2 wherein said electrical circuit is contained within a box (50), with the two terminals (64, 66) external to the box (50) and connected with the electrical circuit by way of extension leads (60, 62) connected to plug-in connectors, said box (50) having corresponding sockets (56, 58) to receive said plug-in connectors, said circuit including a series ammeter (52) and a variable resistor (70).

5. An apparatus according to claims 1 or 2 wherein said electrical circuit is contained within a box (30) adapted to be hand-held, and wherein at least one of said terminals is located on one or more sides of said box.

6. An apparatus according to claim 5 wherein the second of said terminals is located at the opposite side of said box.

7. An apparatus according to claim 5 wherein said first terminal (32) is the positive terminal and is integral with said box and said second terminal is the negative terminal and is positioned at the end of an extendable arm (36).

8. An apparatus according to claim 7 wherein said extendable arm (36) is adjustable in length.

## Patentansprüche

1. Ein Apparat zur Feststellung von mit Haut überdeckten Stellen eines Tierkörpers bestehend aus:
a) zwei metallischen, elektrisch leitenden Anschlußklemmen (32, 40/64, 66) zum Anlegen an besagten Körper;
b) besagte Anschlußklemmen (32, 40/64, 66) sind Bauteile eines elektrischen Schaltkreises;
c)
(i) die erste (32/66) der beiden Anschlußklemmen (32, 40/64, 66) ist durch einen ersten Widerstand (6) mit der ersten Basiselektrode eines Transistors (10) verbunden; besagter Transistor ist so eingestelt, daß ein Impulsstrom bei Einbzw. Ausschalten desselben erzeugt wird;
(ii) eine zweite Elektrode des besagten Transistors (10) ist mit einem Ende (12) der Primärwicklung eines Transformators (14) verbunden;
(iii) eine Electrode des besagten Transistors (10) ist mit einer Seite einer Batterie (16) verbunden, während die andere seite der Batterie (16) mit einer zwischengeschalteten Anzapfstelle an besagtem Transformator (14) verbunden ist;
(iv) die zweite (40/64) der besagten Anschlußklemmen ist direkt durch Zwischenschalten eines Widerstandes mit dem gegenüberliegenden Ende (24) der besagten Primärwicklung verbunden;
(v) ein Kondensator (26) ist zwischen besagte Basiselektrode und besagte zweite Anschlußklemme geschaltet, wobei sich der Kondensator bei erhöhtem Nennentladestrom entlädt, wenn der elektrische spezifische Widerstand der Haut sinkt, wodurch eine erhöhte Impulsfrequenz des besagten Impulsstroms erzeugt wird;
(vi) ein Anzeigegerät (28) ist über eine Sekundärwicklung des besagten Transformators (14) geschaltet, wobei besagtes Anzeigegerät so eingestellt ist, daß es die Frequenz des besagten Impulsstroms anzeigt.

2. Ein nach Patentanspruch 1 eingestellter Apparat, der eine elektrische Behandlung an besagtem Körper anhand einer therapeutischen Dosierungsstärke eines Impulsstroms ermöglicht.

3. Ein apparat nach Patentanspruch 1 oder 2, bei dem das besagte Anzeigegerät (28) ein Apparat zum Aussenden akustischer Signale auf einer Frequenz des besagten Impulsstroms ist.

4. Ein Apparat nach Patentanspruch 1 oder 2, bei dem besagter elektrischer Schaltkreis in einem Gehäuse (50) liegt, mit dem zwei Anschlußklemmen (64, 66) außerhalb des Gehäuses (50), die mit dem elektrischen Schaltkreis durch Verlängerungskabel (60, 62) mit Steckern verbunden sind, die in die entsprechenden Steckdosen (56, 58) im Gehäuse (50) passen, wobei besagtem Schaltkreis ein Reihenstrommesser (52) und ein Regelwiderstand (70) angegliedert sind.

5. Ein Apparat nach Patentanspruch 1 oder 2, bei dem ein Gehäuse (30), das in der Hand gehalten werden kann, besagten Schaltkreis enthält, und bei dem mindestens eine der besagten Anchlußklemmen für eine oder mehr Seiten des besagten Gehäuses ausgelegt ist.

6. Ein Apparat nach Patentanspruch 5, bei dem die zweite der besagten Anschlußklemmen an der gegenüberliegenden Gehäuseseite liegt.

7. Ein Apparat nach Patentanspruch 5, bei dem die erste Anschlubklemme (32) positiv und in besagtes Gehäuse eingebaut ist, und die besagte zweite Anchlußklemme negativ ist und sich am Ende eines ausfahrbaren Arms (36) befindet.

8. Ein Apparat nach Patentanspruch 7, bei dem die Länge des besagten ausfahrbaren Arms (36) justiert werden kann.

## Revendications

1. Un appareil servant à déterminer les points d'acupuncture du corps d'un animal, consistant en:
a) deux bornes conductrices métalliques (32, 40/64, 66) pour établir le contact avec ledit corps;
b) lesdites bornes (32, 40/64, 66) forment une partie d'un circuit életrique;
c)
(i) la première (32/66) des deux bornes (32, 40/64, 66) est branchée par une première résistance (6) au premier électrode de base d'un transistor (10); ledit transistor est adapté pour générer un courant pulsatoire à l'aide de son interrupteur allumé/éteint;
(ii) un deuxième électrode dudit transistor (10) est connecté à une extrémité (12) de l'enroulement primaire d'un transformateur (14);
(iii) un troisième électrode dudit transistor (10) est connecté à un côté d'une pile (16), l'autre côté de la pile (16) étant connecté à une prise intermédiaire dudit transformateur (14);
(iv) la deuxième (40/64) borne est connectée directement ou par l'intermédiaire d'une résistance à l'extrèmitè opposée (24) dudit enroulement primaire;
(v) un condensateur (26) est relié entre ledit èlectrode de base et la deuxième borne, laquelle est déchargée par le condensateur à une vitesse accrue pendant que la résistivité électrique de la peau diminue, générant ainsi une augmentation de la frèquence du pouls dudit courant pulsatoire;
(vi) un indicateur (28) est branché à l'opposé d'un enroulement secondaire dudit transformateur (14) et adapté pour indiquer la fréquence dudit courant pulsatoire.

2. Un appareil, selon la revendication 1, adapté de façon à fournir un traitement électrique dudit corps sous la forme d'un courant pulsatoire en dose thérapeutique.

3. Un appareil, selon les revendications 1 ou 2, dans lequel ledit indicateur (28) est un appareil servant à émettre des sons à la fréquence dudit courant pulsatoire.

4. Un appareil, selon les revendications 1 ou 2, dans lequel ledit circuit électrique est contenu dans une boîte (50), avec les deux bornes (64, 66) se trouvant à l'extérieur de la boîte (50) et ètant reliées au circuit électrique par l'entremise de fils de rallonge (60, 62) connectès aux prises, ladite boîte (50) ayant les douilles correspondantes (56, 58) pour recevoir lesdites prises, ledit circuit comprendant un ampèremètre en sèrie (52) et une rèsistance variable (70).

5. Un appareil, selon les revendications 1 ou 2, dans lequel ledit circuit èlectrique est contenu dans une boîte (30) adaptée de façon à tenir dans une main et dans lequel au moins l'une des dites bornes est située sur un ou plusieur des côtés de ladite boite.

6. Un appareil, selon la revendicatione 5, dans lequel la deuxième des dites bornes est située du côtè opposè de ladite boîte.

7. Un appareil, selon la revendication 5, dans laquel la première borne (32) est la borne positive et fait partie intégrante de ladite boîte, et la deuxième borne est la borne nègative et est située à l'extrémité d'un levier extensible (36).

8. Un appareil, selon la revendication 7, dont la longueur dudit levier extensible (36) peut être modifiée.
